# EUROPÄISCHE PATENTANMELDUNG

(11) **EP 0 647 453 A1**
(43) Veröffentlichungstag der Anmeldung: **12.04.1995**
(21) Anmeldenummer: 94114838.9
(22) Anmeldetag: 21.09.1994
(51) Int. Cl.: A61M 5/168, A61M 5/172, A61M 5/155

(54) **Verfahren zur Verstellung einer schaltbaren durchflussbegrenzenden Vorrichtung und eine nach dem Verfahren arbeitende Vorrichtung**

(30) Priorität: 08.10.1993 DE 4334247
(71) Anmelder: OPTON Feintechnik Kiel GmbH, D-24106 Kiel (DE)
(72) Erfinder: Baumann, Hans, Dr., D-24223 Raisdorf (DE); Hinrichs, Kai-Jürgen, D-24118 Kiel (DE); Otto, Karl-Heinz, D-24146 Kiel (DE); Peters, Jörg-Roger, D-24241 Schmalstede (DE); Graczyk, Wolfgang, D-24109 Kiel (DE)
(74) Vertreter: Tönnies, Jan G. Boehmert & Boehmert, Anwaltssozietät

(57) **Zusammenfassung**

Die Erfindung betrifft Verfahren zur Verstellung einer schaltbaren durchflußbegrenzenden Vorrichtung (42) zum begrenzten Durchfluß von Flüssigkeiten oder Gasen mittels eines Bedienungsgerätes (32). Dabei ist zwischen der Vorrichtung (42) und dem Bediengerät (32) eine räumliche Distanz vorhanden und zwischen beiden besteht keine körperliche Verbindung.

Erfindungsgemäß erfolgt die zur Verstellung der durchflußbegrenzenden Vorrichtung (42) notwendige Energie durch eine Energieübertragung aus dem körperlich getrennten Bediengerät (32) in die Einrichtung (33) mit der durchflußbegrenzenden Vorrichtung (42). Dabei kann eine Schaltung der durchflußbegrenzenden Vorrichtung (42) nur in der Zeit der Energieübertragung erfolgen.

Die Vorrichtung, welche nach dem Verfahren arbeitet, bestehend aus einer Einrichtung (33) mit durchflußbegrenzenden Vorrichtung (42) und einem externen Bediengerät (32), welches ein Schalten der durchflußbegrenzenden Vorrichtung (42) erlaubt.

Dabei ist wesentlich, daß die durchflußbegrenzende Vorrichtung (42) eine multistabile schaltbare durchflußbegrenzende Vorrichtung ist.

## Beschreibung

Die vorliegende Erfindung betrifft Verfahren für eine schaltbare durchflußbegrenzende Vorrichtung nach dem Oberbegriff des ersten Patentanspruchs und ein Vorrichtung, welches nach einem der Verfahren arbeitet.

Derartige Vorrichtungen werden für die unterschiedlichsten Anwendungsfälle benötigt. Besonders schwierig wird es, wenn diese Vorrichtungen bei ihrem Einsatz nicht mehr ohne weiteres manuell verstellt werden können. Insbesondere für Infusionseinrichtungen werden dabei Vorrichtungen benötigt, welche zudem absolut zuverlässig arbeiten, da von ihrer Funktionsfähigkeit das Leben eines Menschen abhängt. Deshalb wird im folgenden insbesondere auf Vorrichtungen eingegangen, welche sich zum Einsatz in Infusionseinrichtungen eignen. Dies soll die Anwendung der Erfindung auf andere Einsatzgebiete aber nicht ausschließen.

Infusionssysteme, welche in den Körper eines Patienten implantiert werden, sind bereits bekannt. Sie dienen zur kontrollierten Verabreichung von Medikamenten. Implantierbare Infusionseinrichtungen, auch Infusionspumpen oder Medikamentenpumpen genannt, finden Verwendung insbesondere bei folgenden Krankheitsbildern: Spastik-, Schmerz-, Chemotherapie usw.. Sie ermöglichen eine direkte, kontinuierliche Medikamentenverabreichung (z.B. Baclofen, Morphin) in das arterielle oder venöse System, sowie in den epidualen oder intraspiralen Raum des menschlichen Körpers. Die Art der medikamentösen Versorgung erlaubt eine so geringe Dosis (0,5 bis 3 ml pro 24 Stunden), daß die Lebensqualität des Patienten erhalten bleibt und dem Organismus belastende Nebeneffekte, wie sie bei herkömmlicher Therapie (Tabletten, Tropfen, Injektionen) bekannt sind, erspart bleiben.

Implantierbare Infusionseinrichtungen mit einer festvorgegebenen Flußrate haben den Nachteil, daß wenn nach Implantation der Einrichtung sich das Krankheitsbild des Patienten im Laufe der Therapie so verändert, daß ein größerer oder kleinerer Medikamentenbedarf notwendig wird, eine sehr kostenaufwendige Pumpenexplantation und Neuimplantation vorgenommen werden muß, um die Lebensqualität des Patienten wieder herzustellen.

Bei implantierbaren Infusionseinrichtungen ist es außerdem in Verbindung mit bestimmten Therapien erwünscht, die Menge des Medikaments, die in einer Zeiteinheit abgegeben wird, zu verändern.

Implantierbare Infusionseinrichtungen mit einer Batterie als Energiequelle sind teilweise mit einer regelbaren Flußrate ausgestattet und werden elektronisch gesteuert. Diese Infusionseinrichtungen haben im Körper des Patienten eine normale, durch die Lebensdauer des Energiespeichers begrenzte Lebensdauer von ca. 36 Monaten. Danach ist eine Explantation der Pumpe notwendig, um die Energiequelle zu erneuern. Durch die begrenzte Lebensdauer des Energiespeichers (Batterie oder Akkumulator) ist die Funktionsdauer des Implantats somit auf eine relativ kurze Zeit begrenzt. Diese Geräte besitzen außerdem ein sehr aufwendiges und meist als bedienerunfreundlich angesehenes steuergerät, mit dem der Arzt Flußratenänderungen und - zyklen per Programm neu vorgeben kann.

Rechtliche Bestimmungen, zum Beispiel in Deutschland, können aber eine Wiederimplantation verbieten, obwohl eine mehrmalige Nutzung der aufwendigen Infusionseinrichtungen durchaus möglich wäre.

Auch aus Kostengründen ist somit momentan die Zielgruppe von Patienten sehr begrenzt und umfaßt hauptsächlich die Schmerztherapie im finalen Stadium.

Bei einer fortgeschrittenen Version einer Infusionseinrichtung ohne elektrischen Energiespeicher können verschiedene Drosselstrecken miteinander kombiniert werden (DE-OS 41 23 091), wodurch es möglich ist, verschiedene Flußraten einzustellen. Dies wird durch ein mechanisches bistabiles Element erreicht, welches vor der Implantation mit einem Schlüssel von Hand eingestellt wird. Die Verstellung kann auch nach der Implantation durch einen kleinen operativen Eingriff erfolgen. Durch einen kleinen Schnitt werden dabei die Elementverstellungen von außen zugänglich und das Implantat als solches kann im Körper verbleiben.

Aus der DE-OS 32 47 232 ist ein Infusionssystem zur medikamentösen Versorgung bekannt, bei dem das implantierte Infusionsgerät eine Betriebsinformations-Speichereinrichtung aufweist, welche die gespeicherten Daten an eine Fernmeßeinrichtung außerhalb des Körpers des Patienten mit implantiertem Infusionsgerät übertragen kann. Diese Fernmeßeinrichtung und das Infusionsgerät weisen einen Sender und einen Empfänger auf, so daß von einer körperexternen Befehlsgebereinrichtung ein Befehl zu einer ferngesteuerten Betriebsbereitschaft zum Infusionsgerät übermittelt werden kann. In dem System können diverse Betriebsdaten von außen abgefragt werden und die Infusionsraten von außen verändert werden. Dazu besitzt das Infusionsgerät diverse Sensoren zur Erfassung der Betriebsdaten und eine Pumpe, welche im Impulsbetrieb arbeitet.

Aus der DD-PS 29 30 55 ist eine elektromagnetisch gesteuerte bistabile Einrichtung für implantierbare, Treibgas betriebene Infusionspumpen bekannt, welche eine bistabile Einrichtung mit entgegengesetzt anliegenden Betriebszuständen besitzt. Die Energiezuführung erfolgt dabei induktiv von außen. Die bistabile Einrichtung erlaubt dabei nur die beiden Betriebszustände Aus und Ein.

Aus der EP-PS 0 019 814 ist eine Steuereinrichtung für Infusionsgeräte bekannt, bei welcher Steuersignale in das implantierte Gerätegehäuse des Infusionsgerätes codiert werden.

Aus der EP-OS 0 110 117 ist ein implantierbares Mikroinfusionspumpensystem bekannt, welches eine Pumpe mit Impulsbetrieb beinhaltet.

Aus der EP-PS 0 031 850 ist ein implantierbares magnetgesteuertes System für die Infusion von Arzneimitteln bzw. Medikamenten in einen lebenden Körper mit einer druckbetätigten Arzneimittelabgabevorrichtung bekannt, welches ein bewegbares bistabiles Element besitzt.

Aus der EP-PS 0 039 124 ist ein implantierbares Infusionsgerät bekannt, welche einen Durchflußbegrenzer aufweist.

Aus der EP-PS 0 128 703 ist eine Mikropumpe zur Implantation bekannt, welche als Strömungssteuerung ein Ein/Aus-Element besitzt, welches normaler Weise geschlossen ist und von einem externen Elektromagneten betätigt wird.

Es ist die Aufgabe der Erfindung ein Verfahren zur Verstellung einer schaltbaren durchflußbegrenzenden Vorrichtung und ein danach arbeitende Vorrichtung zu realisieren, welche keine zeitliche Nutzungsbegrenzung durch einen elektrischen Energiespeicher aufweist und bei welcher eine Veränderung der Betriebsstellung, und damit eine Veränderung der Flußrate durch die Vorrichtung ohne direkten körperlichen Kontakt mit der Vorrichtung möglich ist.

Diese Aufgabe wird durch die Merkmale des kennzeichnenden Teils des ersten Patentanspruchs gelöst.

Durch die erfindungsgemäße Verfahrensweise der Verstellung wird im Betrieb (insbesondere auch von Infusionseinrichtungen) eine optimale Verstellsicherheit erreicht, ohne daß man auf die vorteilhafte Verstellmöglichkeit als solche verzichten muß, bzw. ohne daß für die Verstellung ein direkter körperlicher Kontakt mit der Vorrichtung notwendig ist.

Man erhält alle Vorteile der elektronisch gesteuerten Vorrichtungen, ohne daß es zu einer unbeabsichtigten, unkontrollierten Verstellung der Flußrate kommen kann.

Die Variationsbreite, welche einem zur Einstellung der verschiedenen Flußraten zur Verfügung steht und die damit verbundene Anzahl der notwendigen durchflußbegrenzenden Vorrichtungen in einer räumlich begrenzten Einrichtung läßt sich durch multistabile durchflußbegrenzende Ventile optimieren.

Die Verstellung der durchflußbegrenzenden Vorrichtung läßt sich noch sicherer machen, wenn die externe Energieeinspeisung nur zeitweise erfolgt. Die Energieeinspeisung sollte dabei vorteilhafterweise immer dann erfolgen, wenn eine Verstellung der durchflußbegrenzenden Vorrichtung vorgenommen werden soll.

Wenn die Einstellung von unterschiedlichen Flußraten (z. B. für Medikamente aus einem Vorratsbehälter) mittels nach der durchflußbegrenzenden Vorrichtung angeordneten, unterschiedlichen Drosselstrecken erfolgt, kann sich nach einer Einstellung der durchflußbegrenzenden Vorrichtung die Flußrate bis zur nächsten Verstellung der durchflußbegrenzenden Vorrichtung nicht mehr ändern. Damit sorgt auch diese Vorgehensweise für eine erhöhte Sicherheit beim Betrieb. Dies ist insbesondere für Infusionseinrichtungen sehr wichtig.

Die multistabile durchflußbegrenzende Vorrichtung kann vorteilhalfterweise dadurch realisiert werden, daß ein Kolben im Innern der durchflußbegrenzenden Vorrichtung bewegt wird, daß der Kolben zwei stabile Endlagen und daß ein dritter lagestabiler Zustand des Kolbens durch die Kombination von magnetischer Haltekraft und rückstellender Federkraft erreicht wird.

Für die Betriebssicherheit und Lebensdauer ist es sehr vorteilhaft, wenn die durchflußbegrenzende Vorrichtung stromlos in ihren stabilen Stellungen verbleibt.

Es ist vorteilhaft, wenn die externe Energieeinspeisung induktiv erfolgt. Diese Energieeinspeisung ist kostengünstig und benötigt relativ wenig Platz zu ihrer Realisierung. Dies ist insbesondere für Infusionseinrichtungen wichtig.

Sehr vorteilhaft ist es, wenn eine Datenübertragung in die und aus der Einrichtung mit der durchflußbegrenzenden Vorrichtung existiert. Dazu muß sich in der Einrichtung und in einem externen Bediengerät eine Datenübertragungseinrichtung befinden. Dies ermöglicht eine Datenübertragung von im Innern der Einrichtung z.B. im EEPROM zwischengespeicherten Daten zum Bediengerät. Es können aber auch andere Daten, welche außerhalb der Einrichtung mit der durchflußbegrenzenden Vorrichtung ermittelt und zu dieser weitergeleitet wurden, so nach außen übertragen werden. Dies bedeutet für eine implantierte Infusionseinrichtung, daß die Daten aus dem Inneren des Patientenkörpers nach außerhalb des Körpers des Patienten übertragen werden.

Bei dem Einsatz der durchflußbegrenzenden Vorrichtung in einer Infusionseinrichtung ist es vorteilhaft, wenn die Füllstandswerte des Vorratsbehälters in der Infusionseinrichtung gemessen werden und diese Daten in einem elektronischen Speicher in der Infusionseinheit zwischengespeichert werden. Als elektronische Speicher bieten sich insbesondere EEPROM's an, da bei diesen keine Energie zur Aufrechterhaltung des Speicherinhaltes notwendig ist.

Als flüssigkeitsfördernde Einrichtung eignet sich in der Infusionseinrichtung insbesondere ein Gasdruckbehälter, welcher auf den Vorratsbehälter einen Druck in Richtung der durchflußbegrenzenden Vorrichtung ausübt. Dieser Gasdruckbehälter hat den Vorteil der sehr hohen Betriebssicherheit bei relativ kleinem Raumbedarf.

Vorteilhafte Ausgestaltungen der durchflußbegrenzenden Vorrichtung sind in den Patentansprüchen und insbesondere in den Figurenbeschreibungen erläutert.

Sehr vorteilhaft ist es auch, wenn die Einrichtung mit der durchflußbegrenzenden Vorrichtung eine elektronische Schaltung besitzt. Diese dient insbesondere zur Datenübertragung, gegebenenfalls zur Datenermittlung, gegebenenfalls zur Datenspeicherung und gegebenenfalls auch zur Vorverarbeitung der ermittelten Daten.

Die vorteilhafte Ausgestaltung der elektronischen Schaltung ist in den Patentansprüchen und insbesondere in der Figurenbeschreibung näher erläutert.

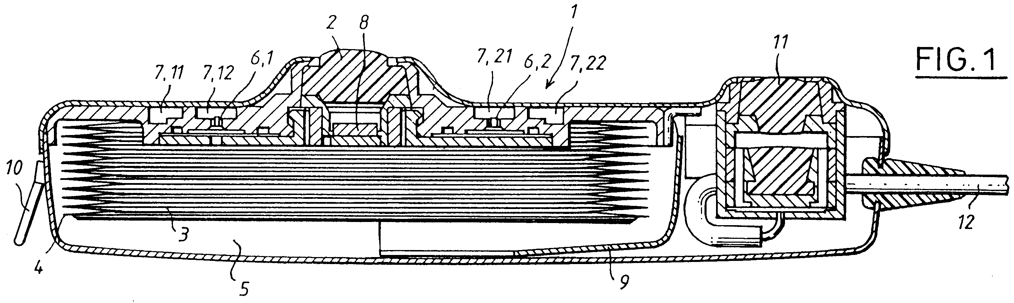

Die Erfindung wird im folgenden anhand der Ausführungsbeispiele in den Zeichnungen Figur 1 bis Figur 6 näher erläutert, wobei weitere erfindungswesentliche Merkmale im Zusammenhang erläutert werden. Dabei wird beispielhaft der Einsatz der durchflußbegrenzenden Vorrichtung in einer Infusionseinrichtung beschrieben, wobei die Erfindung aber nicht auf die Nutzung der Vorrichtung in einer derartigen Einrichtung begrenzt sein soll.

Dabei zeigen:
- Fig. 1: eine Infusionseinrichtung mit einer durchflußbegrenzenden Vorrichtung gemäß der Erfindung;
- Fig. 2: einen Schnitt durch die durchflußbegrenzende Vorrichtung;
- Fig. 3: eine Aufsicht eines Schnittes durch die Vorrichtung aus Figur 2;
- Fig. 4: das Federelement aus der Vorrichtung in Aufsicht;
- Fig. 5: das Federelement aus Fig.4 in 3D-Darstellung; und
- Fig. 6: ein Schema der im Bediengerät und in der Infusionseinrichtung enthaltenen Komponenten.

Die in der Figur 1 dargestellte Infusionspumpe (1) besitzt ein Septum (2), durch welches ein Medikament mit einer Spritze (in der Figur nicht dargestellt) in den Vorratsbehälter (3) durch die Bauchdecke eingefüllt werden kann, wenn die Infusionspumpe (1) in den Körper eines Patienten implantiert wurde. Der Vorratsbehälter (3), welcher als Medikamentenkammer dient, wird durch einen Faltenbalg (4) begrenzt, der bei Befüllung expandiert. Dieser Faltenbalg (4) besteht aus Titan. Der nahezu konstante Überdruck des Treibgases im Gasdruckbehälter (5) übt auf diesen Faltenbalg (4) einen Druck aus, wodurch es im Laufe der Zeit zu einer Entleerung des Medikamentes im Vorratsbehälters (3) kommt. Der Gasdruckbehälter (5) wird seitlich durch eine Trennwand (9) begrenzt und ist so dimensioniert, daß das Gas während der ganzen Dauer der Entleerung der Vorratsbehälters (3) auf diesen einen nahezu konstanten Druck ausübt.

Seitlich ist an dem Gehäuse der Infusionspumpe (1) eine Nahtöse (10) angebracht, an welcher die Infusionspumpe (1) an das umgebende Gewebe festgenäht wird, damit die Infusionspumpe (1) ihre Lage während der Dauer der Implantation nicht zu sehr verändert und dadurch dem Träger Schmerzen verursacht.

Die Drosselstrecken (7) begrenzen die Medikamentenmenge, welche pro Zeiteinheit aus dem Vorratsbehälter abgegeben werden kann. Diese Drosselstrecken (7) beginnen hinter dem Ventil (6), welches die Drosselstrecken (7) für den Medikamentenfluß öffnet oder verschließt. Die Drosselstrecken (7) enden alle in einem Bolus-Septum (11) und haben dort über einen Katheter (12) einen Ausgang in das Körperinnere. In dieses Bolus-Septum (11) kann auch vom Arzt direkt eingespritzt werden, um eine zusätzliche Medikamentengabe dem Träger der Infusionspumpe (1) zu verabreichen.

Das Septum (2) und das Bolus-Septum (11) ragen aus dem Gehäuse der Infusionspumpe (1) heraus, so daß der Arzt bei der Suche nach den beiden Septa (2, 11) diese ertasten kann.

Im Septum (2) sorgt ein Nadelstop (8) dafür, daß bei der Auffüllung des Vorratsbehälters (3) die Injektionsnadel (in der Zeichnung nicht eingezeichnet) nicht beschädigt wird.

Der Austritt des Medikaments aus der Infusionspumpe (1) erfolgt über die dem Ventil (6) nachgeschaltete Drosselstrecken (7), so daß eine konstante Flußrate erreicht wird. In jedes der beiden Ventile (6.1, 6.2) geht ein Zufluß aus dem Vorratsbehälter (3) und aus jedem Ventil (6.1, 6.2) gehen je zwei Drosselstrecken (7.11, 7.12; 7.21, 7.22) heraus. Die Flußrate hängt neben dem Gasdruck dabei primär von der Länge der Drosselstrecken (7) und deren Kapillardurchmesser ab.

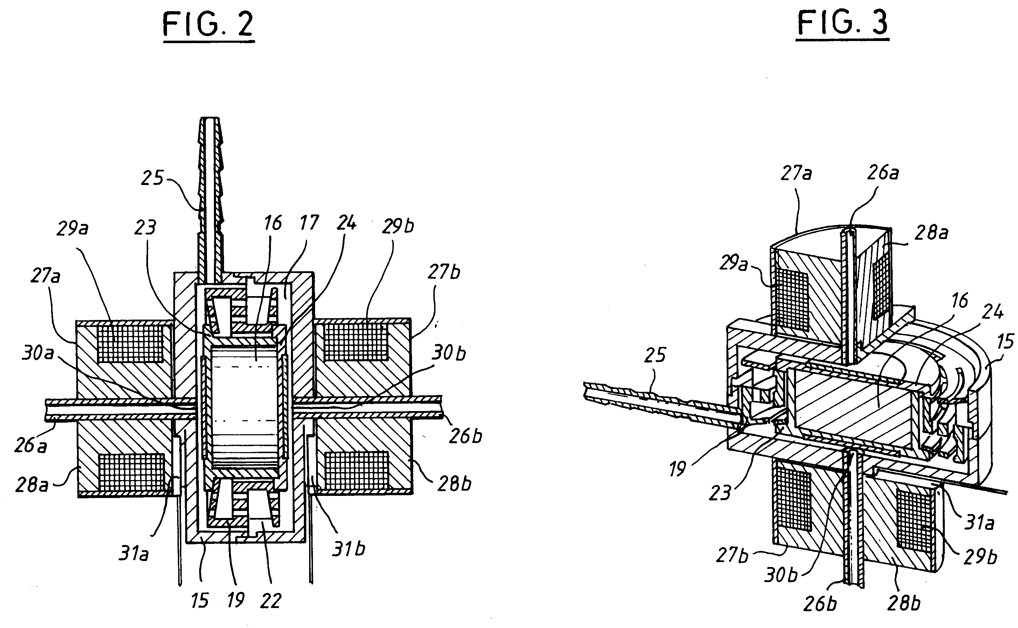

Verwendet man mehrere Drosselstrecken (7), dann kann man durch Parallel- und/oder Serienschaltung diverser Drosselstrecken (7) unterschiedliche Flußraten erzielen. Die Umschaltung erfolgt dabei durch ein tristabiles elektromechanisches Mikroventil (6). Dieses Ventil (6) wird in den nachfolgenden Figuren 2, 3 und 4 näher erläutert.

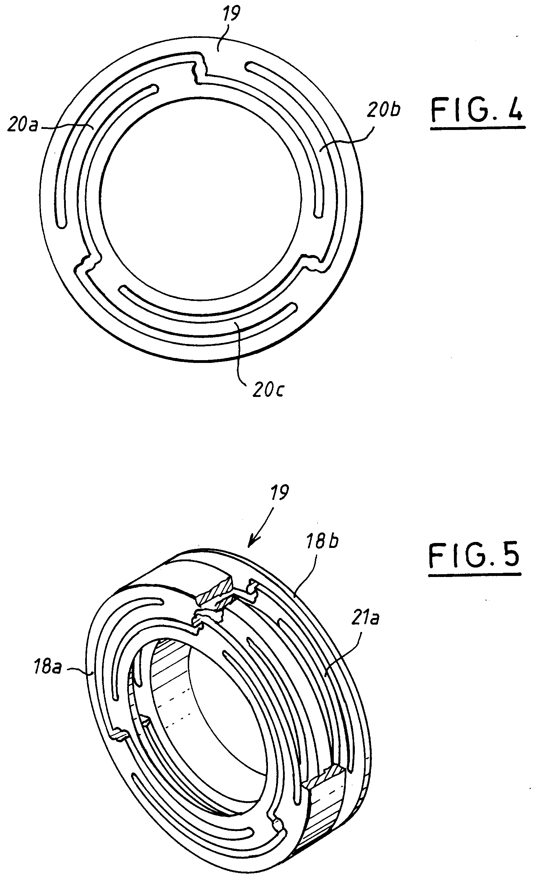

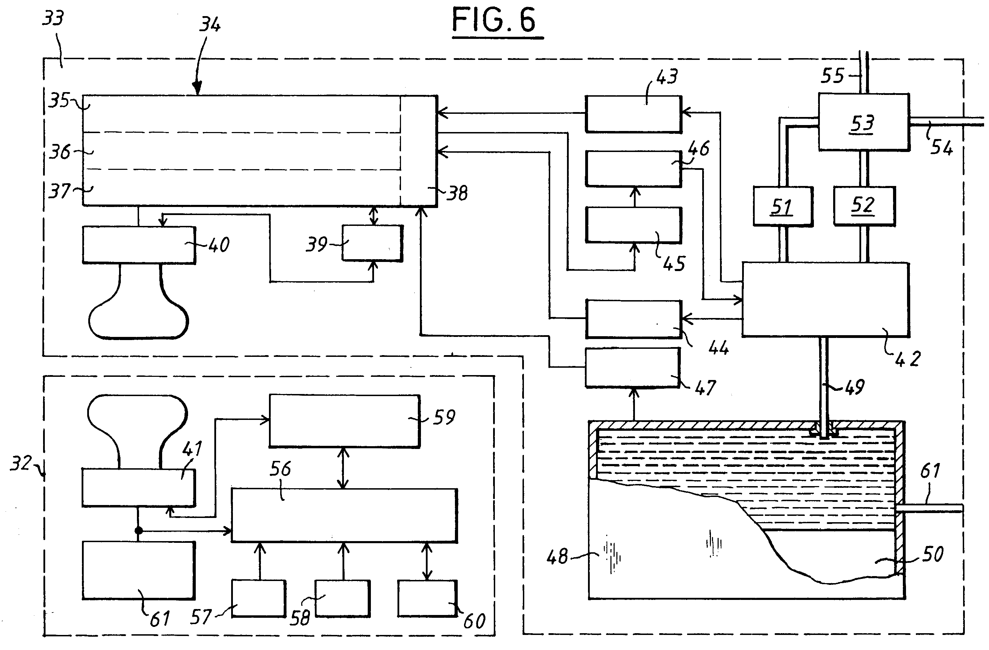

In der Figur 2 ist ein Schnitt durch ein tristabiles elektromechanisches Ventil (15) dargestellt und in Figur 3 die Ansicht eines Schnittes durch das Ventil (15). Der Kolben (16) besteht aus einem permanentmagnetischen Material (z.B. Vacodym) und ist mit einem gegenüber den eingesetzten Medikamenten kompatiblen (d.h. nicht reagierenden) Material (z.B. Titan) der Kapselung (23, 24) gekappselt. Die Befestigung des Kolbens (16) besteht aus einem Federelement (19) (siehe Figur 4 und 5).

Das Federelement (19) aus Fig. 2 zentriert und führt zugleich den Kolben (16) innerhalb der Ventilkammer (17).

Die in zwei Ebenen (18a, 18b) verlaufenden Blattfedern aus Fig. 5 erzeugen eine jeweils zur Mitte hin gerichtete Vorspannung, die eine stabile Mittenlage des Kolbens (16) ermöglicht. Die Vorspannungen werden durch Verformung des Federelementes (19) bei der Montage erzeugt. Der Montagering (22) spreizt einen Teil des Federelementes (21a, b, c) und erzeugt so eine Kraftkomponente auf den Kolben (16) in positiver Z-Richtung. Durch die Kapselung (23, 24) des Kolbens aus Fig. 2 werden die Blattfedern (20a, b, c) innen vorgespannt, wodurch eine Kraft in negativer Z-Richtung erzeugt wird. Bei Bewegung des Kolbens in negativer bzw. positiver Z-Richtung, werden richtungsabhängig jeweils Blattfedern der einen Ebene entlastet und in der anderen Ebene weiter gespannt oder umgekehrt.

Die Ventilkammer (17) besitzt einen seitlichen Zufluß (25) und zwei sich gegenüberliegende, stirnseitig angeordnete zentrische Abflüsse (30a, 30b), an welchen jeweils ein Kapillaranschluß (26a, 26b) zu den Drosselstrecken (7) angebracht ist.

Stirnseitig auf die Ventilkammer (15) aufgesetzt befindet sich je ein Elektromagnet (27a, 27b) mit einem Spulenkörper (28a, 28b) aus einem weichmagnetischen Material. Durch richtige Polung der beiden Spulen (29a, 29b) kann der permanentmagnetische Kolben (16) aus seiner, durch die Blattfedern (20a, b, c; 21a, b, c) definierten Mittelstellung, nach + Z- bzw. - Z-Richtung bewegt werden. Wenn der Kolben (16) vollständig in eine Randlage angezogen ist, verschließt sich einer der beiden Abflüsse (30a, 30b). Nach dem Ausschalten des Stroms bleibt diese Stellung des Ventils (15) erhalten. Die erforderlichen Haltekräfte erzeugt der permanentmagnetische Kolben (16) und die durch ihn erzeugte Magnetisierung des jeweiligen Spulenkörpers (28a, 28b) der Elektromagnete (27a, 27b). Die Dimensionierung ist dabei so vorzunehmen, daß die permanentmagnetische Haltekraft größer ist als die Rückstellkraft der Blattfedern (20a, b, c; 21a, b, c).

Die jeweilige Stellung des Ventils (15) wird über für Magnetfelder empfindliche Sensoren (31a, 31b) festgestellt, die stirnseitig an der, der Ventilkammer (17) zugewandten Seite der Elektromagneten (27a, 27b) eingebaut sind. In den Endlagen stellt sich dabei bei dem betreffenden Sensor (31a, 31b) eine maximale Feldstärke ein.

In der durch die Blattfedern (20a, b, c; 21a, b, c) definierten Mittellage (M) ist die Feldstärke für beide Endlagensensoren (31a, 31b) etwa gleich. Diese Mittelstellung kann durch eine dynamisch gesteuerte Verstellung des Ventils (15) erreicht werden. Durch die eingebaute Sensorik (31a, 31b) ist es möglich den Kolben (16) durch eine geeignete Ansteuerelektronik (z.B. PI-Regler, bei welchem in der Mittelstellung der Fluß durch beide Magnetfeldsensoren gleich ist:
Φ₁ = Φ₂ ==> Φ₁ - Φ₂ = 0 Sollwert für Regler) in die Mittellage zu bringen. Nach dem Ausschalten der Elektromagnete (27a, 27b) bleibt dieser Zustand erhalten, da die Federkraft die sich dann kaum unterscheidenden magnetischen Anziehungskräfte dominiert.

Der Kolben (16) kann durch die Orientierung des Stroms durch die Spulen (29a, 29b) der beiden Elektromagnete (27a, 27b) in seine stabilen Lagen gesteuert werden. Dabei wird bei einer Endlage des Kolbens (16) jeweils eine der beiden Kapillarenanschlüsse (26a, 26b) an den beiden Abflüsse (30a, 30b) geschlossen oder geöffnet. In der Mittellage sind beide Kapillarenanschlüsse (26a, 26b) geöffnet.

In Figur 6 ist schematisch das extrakorporale Bediengerät (32) und die implantierte Infusionseinrichtung (33) dargestellt.

Die Infusionseinrichtung (33) besitzt eine elektronische Steuereinrichtung (34), welche alle Aktivitäten in der Infusionseinrichtung (33) steuert. Diese Steuereinrichtung (34) besteht im wesentlichen aus einem EEPROM (35), einem Single-Chip-Computer (36), einer seriellen Schnittstelle (37) (auch eine parallele Schnittstelle wäre denkbar, aber unvorteilhaft) und einem Port (38), an welchem diverse Treiber und Sensoren angeschlossen werden können.

Die serielle Schnittstelle (37) ist mit einem Modulator/Demodulator (39) für die Absorptionstelemetrie (40) verbunden. Diese Absorptionstelemetrie (40) besteht aus einem NF/HF-Wandler und dient zur Kommunikation der Infusionseinrichtung (33) mit dem extrakorporalen Bediengerät (32). Die gleiche Antenne dient sowohl der Energieübertragung, als auch der bidirektionalen Datenübertragung.

In dem Bediengerät (32) ist eine Spule (32a) enthalten, über welchem in die Infusionseinrichtung (33) induktiv Energie übertragen werden kann. Die übertragene Energie dient zum Betrieb des Single-Chip-Computers (36), der Sensoren, der Treiber und des Ventils (42). Die Energieeinkopplung erfolgt nur in Gegenwart des extrakorporalen Bediengerätes (32).

Mit dem Port (38) des Single-Chip-Computer (36) sind zwei Sensoren (43, 44) verbunden, welche die Stellung des Ventils (42) ermitteln. Die Funktionsweise dieser Sensoren (43, 44) ist im Zusammenhang mit Fig. 2, 3, und 4 schon erläutert worden.

Außerdem ist mit dem Port (38) ein Treiber (45) für einen Ventilumschalter (46) verbunden. Durch einen entsprechenden Befehl des Bediengerätes (32), welcher vom Empfänger in der Absorptionstelemetrie (40) empfangen und dem Single-Chip-Computer (36) über die serielle Schnittstelle (37) übermittelt wurde, kann so die Stellung des Ventils (42) verändert werden. Die entsprechenden Ansteuercharakteristika werden dabei vom Single-Chip-Computer (36) erzeugt und der Umschalter (46) entsprechend beeinflußt.

Desweiteren ist ein Füllstandssensor (47) mit dem Port (38) des Single-Chip-Computers (36) verbunden, welcher eine Meldung über den Füllstand, d.h. über die noch vorhandene Medikamentenmenge im Vorratsbehälter (48), an den Microcontroller (36) übermittelt. Der Microcontroller (36) berechnet daraus die noch verfügbare Infusionszeit und überprüft, ob die Flußmenge mit den eingestellten Werten übereinstimmt.

Vom Vorratsbehälter (48) aus führt eine Leitung (49) zum Ventil (42). Der Vorratsbehälter (48) ist als Faltenbalg ausgebildet und steht unter Druck von einem Druckgasspeicher (50). Der durch den Druckgasspeicher (50) auf den Vorratsbehälter (48) ausgeübte Druck treibt das in diesem enthaltene Medikament in Richtung auf das Ventil (42).

Aus dem Ventil (42) führen zwei Drosselstrecken (51, 52) heraus. Die Drosselstrecken (51, 52) sorgen dafür, daß sich der Vorratsbehälter (48) nur kontrolliert entleert. Sie bestehen aus Kapillaren und die pro Stunde durch sie hindurchfließende Flüssigkeitsmenge ist abhängig von der Druckdifferenz über den Drosselstrecken (51, 52), von deren Kapillarlänge und vom Kapillardurchmesser der einzelnen Drosselstrecke (51 52), wobei dieser Durchmesser für jede Drosselstrecke (51, 52) unterschiedlich sein kann. Das Ventil (42) öffnet beide oder nur eine Drosselstrecke (51, 52). Solange die Stellung des Ventils (42) nicht verändert wird, bleibt somit die abgegebene Flüssigkeitsmenge pro Stunde konstant.

Die beiden Drosselstrecken (51, 52) enden im Bolus (53), an dessen Ausgang sich ein Katheter (54) ins Körperinnere des Trägers der implantierten Infusionseinrichtung (33) befindet. In den Bolus (53) kann aber auch vom Arzt direkt mit einer Injektionsnadel (55) ein Medikament eingespritzt werden.

Das Bediengerät (32) besitzt einen Trafo (41) als ein Bestandteil der Absorptionstelemetrie (40) zur induktiven Übertragung von Energie zur Infusionseinrichtung (33). Dieser Trafo (41) wird gespeist durch eine Batterie (61). Die Batterie (61) ist auch mit einem Single-Chip-Computer (56) verbunden.

Ein Bediener, dies wird in der Regel ein Arzt sein, kann über eine Tastatur (57) oder über Funktionstasten (58) die von dem Single-Chip-Computer (56) in der Infusionseinrichtung (33) ermittelten Betriebsdaten aus der Infusionseinrichtung (33) abfragen oder eine Verstellung des Ventils (42) in der Infusionseinrichtung (33) veranlassen. Dazu besitzt das Bediengerät (32) eine Datenkommunikationseinrichtung (59), welche aus einem Modulator und Demodulator für die Absorptionstelemetrie (40) besteht. Über eine LCD-Anzeige (60) können alle übertragenen Betriebsparameter aus der Infusionseinrichtung (33) sowie die an diese abgesandten Befehle angezeigt werden.

Das Bediengerät (32) erfüllt somit zwei Aufgaben. Zum einen wird vom Bediengerät (32) aus in die implantierte Infusionseinrichtung (33) induktiv Energie übertragen, wenn man das Bediengerät (32) in die Nähe der Infusionseinrichtung (33) bringt (Bauchdecke). Wenn die Energie eingekoppelt ist, kann man über eine Absorptionstelemetrie, welche sich sowohl im Bediengerät (32) als auch in der Infusionseinrichtung (33) befindet, vom Bediengerät (32) aus das Ventil (42) steuern. In der Infusionseinrichtung (33) sitzt ein Single-Chip-Computer (36), welcher die Kommunikation mit dem Bediengerät (32) realisiert und welcher das in der Infusionseinrichtung (33) befindliche interne tristabile Ventil (42) nach Vorgabe schaltet und dessen Funktion kontrollieren kann.

In der Infusionseinrichtung (33) ist keine elektrische Energie gespeichert, um das Ventil (42) zu schalten. Damit das in der Infusionseinrichtung (33) enthaltene Ventil (42) geschaltet werden kann, muß in die Infusionseinrichtung (33) induktiv Energie übertragen werden.

Entfernt man das Bediengerät (32), dann wird die induktive Kopplung unterbrochen und die implantierte Infusionseinrichtung (33) wird stromlos, so daß eine Verstellung des Ventils (42) nicht erfolgen kann. Die eingestellte Flußrate bleibt jedoch erhalten, da das oder die hintereinander oder parallel geschalteten tristabilen Ventile (42) in ihrer stabilen Betriebslage verbleiben. Durch die Verwendung von mehreren Ventilen (42) kann man dabei die Variationsmöglichkeit der Flußrate sehr erhöhen.

Wenn die Infusionseinrichtung (33) aktiviert ist (d.h. von dem extrakorporalen Bediengerät (32) Energie übertragen wird), kann der Füllstand mittels eines Sensors (47) gemessen werden. Aus dem Füllstand und der eingestellten Flußrate kann dann z.B. die verbleibende Restzeit bis zur nächsten Befüllung dem Patienten an der Anzeige (60) des Bediengerätes (32) angezeigt werden. Die Kontrolle der Flußrate kann dabei vorteilhafterweise über gespeicherte Füllstandswerte ermittelt werden. Als Speicher (35) bieten sich hier besonders EEPROM's an, welche die Füllstandswerte permanent speichern.

Aus der Verstellbarkeit und der Füllstandsmessung und den daraus resultierenden Möglichkeiten ergibt sich eine Flexibilität, welche sonst den elektronischen Infusionseinrichtungen vorbehalten war, ohne daß man den Nachteil der äußerst begrenzten Lebensdauer bei Batteriebetrieb akzeptieren muß.

Durch die erfindungsgemäße Realisierung der Energieeinkopplung wird eine absolute Störunempfindlichkeit gegen äußere Störeinflüsse des letztendlich elektrisch gesteuerten Ventils (42) erreicht. Dagegen besteht bei Infusionseinrichtungen, bei welchen die Verstellung des Ventils durch den Strom einer Stromspeicherungseinrichtung (d.h. mit einer permanenten elektrischen Versorgung, z.B. eine Batterie) erfolgt, immer die Gefahr, daß diese Infusionseinrichtungen durch Störeinflüsse von außen umprogrammiert werden.

In der erfindungsgemäßen Infusionseinrichtung (33) wird eine doppelte Sicherheit erzielt. Zum einen wird die Infusionseinrichtung (33) im Normalzustand passiv. Zum anderen wird aus Sicherheitsgründen die Datenübertragung mittels Absorptionstelemetrie durchgeführt. Durch eine zusätzliche Kodierung der zu übertragenden Daten und Befehle wird zusätzliche Sicherheit gegenüber Störimpulsen realisiert. Hinzu kommt, daß die Absorptionstelemetrie nur im Nahfeld wirksam ist.

## Patentansprüche

1. Verfahren zur Verstellung einer schaltbaren durchflußbegrenzenden Vorrichtung zum begrenzten Durchfluß von Flüssigkeiten oder Gasen mittels eines Bedienungsgerätes, wobei zwischen der Vorrichtung und dem Bediengerät eine räumliche Distanz vorhanden ist und zwischen beiden keine körperliche Verbindung besteht, dadurch gekennzeichnet, daß die zur Verstellung der durchflußbegrenzenden Vorrichtung (6.1, 6.2, 6a.1, 6a.2, 15, 42) notwendige Energie durch eine Energieübertragung aus dem körperlich getrennten Bediengerät (32) in die Einrichtung (1, 33) mit der durchflußbegrenzenden Vorrichtung (6.1, 6.2, 6a.1, 6a.2, 15, 42) erfolgt und daß eine Schaltung der durchflußbegrenzenden Vorrichtung (6.1, 6.2, 6a.1, 6a.2, 15, 42) nur in der Zeit der Energieübertragung erfolgen kann.

2. Verfahren zur Verstellung einer schaltbaren durchflußbegrenzenden Vorrichtung nach Anspruch 1, dadurch gekennzeichnet, daß die durchflußbegrenzenden Vorrichtung (6.1, 6.2, 6a.1, 6a.2, 15, 42) in einer Infusionspumpe (1, 33) zur Implantation in den Körper eines Patienten zur medikamentösen Behandlung des Patienten verwendet wird, daß die Infusionspumpe (1, 33) mindestens eine Einstichstelle zum Auffüllen eines Vorratsbehälter (3, 48) zum Speichern eines gewählten Medikaments aufweist, daß eine flüssigkeitsfördernde Einrichtung (5, 50) zum Transport des Medikaments vom Vorratsbehälter (3, 48) zur durchflußbegrenzenden Vorrichtung (6.1, 6.2, 6a.1, 6a.2, 15, 42) in der Infusionspumpe (1, 33) verwendet wird und daß mindestens ein Vorrichtung (7, 7.11, 7.12, 7.21, 7.22, 51, 52) zum begrenzten Einleiten des aus dem Vorratsbehälter (3, 48) kommenden, in diesem gespeicherten, ausgewählten Medikaments in den Körper nach der schaltbaren durchflußbegrenzenden Vorrichtung (6.1, 6.2, 6a.1, 6a.2, 15, 42) aufweist.

3. Verfahren zur Verstellung einer schaltbaren durchflußbegrenzenden Vorrichtung nach einem der Ansprüche 1 oder 2, dadurch gekennzeichnet, daß die durchflußbegrenzende Vorrichtung (6.1, 6.2, 6a.1, 6a.2, 15, 42) aus einem Ventil besteht und das Ventil (6.1, 6.2, 6a.1, 6a.2, 15, 42) mehrere stabile Betriebszustände besitzt.

4. Verfahren zur Verstellung einer schaltbaren durchflußbegrenzenden Vorrichtung nach einem der Ansprüche 1 bis 3, dadurch gekennzeichnet, daß die externe Energieeinspeisung nur zeitweise erfolgt.

5. Verfahren zur Verstellung einer schaltbaren durchflußbegrenzenden Vorrichtung nach einem der Ansprüche 1 - 4, dadurch gekennzeichnet, daß die Einstellung unterschiedlicher Flußraten mittels nach der durchflußbegrenzenden Vorrichtung (6.1, 6.2, 6a.1, 6a.2, 15, 42) angeordneten, unterschiedlichen Drosselstrecken (7, 7.11, 7.12, 7.21, 7.22, 51, 52) erfolgt.

6. Verfahren zur Verstellung einer schaltbaren durchflußbegrenzenden Vorrichtung nach einem der Ansprüche 1 - 5, dadurch gekennzeichnet, daß ein Kolben (16) im Innern der durchflußbegrenzenden Vorrichtung (6.1, 6.2, 6a.1, 6a.2, 15, 42) bewegt wird, daß der Kolben (16) zwei stabile Endlagen in dem Inneren der durchflußbegrenzenden Vorrichtung (6.1, 6.2, 6a.1, 6a.2, 15, 42) besitzt und daß ein dritter lagestabiler Zustand des Kolbens (16) durch die Kombination von magnetischer Haltekraft und rückstellender Federkraft erreicht wird.

7. Verfahren zur Verstellung einer schaltbaren durchflußbegrenzenden Vorrichtung nach einem der Ansprüche 1 - 6, dadurch gekennzeichnet, daß die durchflußbegrenzende Vorrichtung (6.1, 6.2, 6a.1. 6a.2, 15, 42) stromlos in einer ihrer stabilen Stellungen verbleibt.

8. Verfahren zur Verstellung einer schaltbaren durchflußbegrenzenden Vorrichtung nach einem der Ansprüche 1 - 7, dadurch gekennzeichnet, daß die externe Energieeinspeisung induktiv erfolgt.

9. Verfahren zur Verstellung einer schaltbaren durchflußbegrenzenden Vorrichtung nach einem der Ansprüche 1 - 8, dadurch gekennzeichnet, daß sich in der Einrichtung (1, 33) und in dem Bediengerät (32) eine Datenübertragungseinrichtung (40) befindet und daß eine Datenübertragung von im Inneren der Infusionseinrichtung (1, 30) zwischengespeicherten Daten zum Bediengerät (32) erfolgt.

10. Verfahren zur Verstellung einer schaltbaren durchflußbegrenzenden Vorrichtung nach Anspruch 1 - 9, dadurch gekennzeichnet, daß die Energieübertragung induktiv mittels Absorptionstelemetrie (40) erfolgt.

11. Verfahren zur Verstellung einer schaltbaren durchflußbegrenzenden Vorrichtung nach Anspruch 2, dadurch gekennzeichnet, daß die Füllstandswerte des Vorratsbehälters (3, 48) mit einem Sensor (47) gemessen werden und daß die gemessenen Füllstandswerte in einem Speicher (35) in der Infusionseinrichtung (1, 30) zischengespeichert werden.

12. Vorrichtung, welche nach einem der Verfahren in den Ansprüchen 1 - 11 arbeitet, bestehend aus einer Einrichtung (1, 33) mit durchflußbegrenzenden Vorrichtung (6.1, 6.2, 6a.1. 6a.2, 15, 42) und einem externen Bediengerät (32), welches ein Schalten der durchflußbegrenzenden Vorrichtung (6.1, 6.2, 6a.1. 6a.2, 15, 42) erlaubt, dadurch gekennzeichnet, daß die durchflußbegrenzende Vorrichtung (6.1, 6.2, 6a.1, 6a.2, 15, 42) eine multistabile schaltbare durchflußbegrenzende Vorrichtung (6.1, 6.2, 6a.1, 6a.2, 15, 42) ist.

13. Vorrichtung nach Anspruch 12, dadurch gekennzeichnet, daß die Ausgänge der durchflußbegrenzenden Vorrichtung (6.1, 6.2, 6a.1, 6a.2, 15, 42) jeweils mit einer Drosselstrecke (7, 7.11, 7.12, 7.21, 7.22, 51, 52) verbunden sind.

14. Vorrichtung nach einem der Ansprüche 12 oder 13, dadurch gekennzeichnet, daß die durchflußbegrenzende Vorrichtung (6.1, 6.2, 6a.1, 6a.2, 15, 42) in ihrem Inneren in einer Kammer (17) einen Kolben (16) enthält und daß die Lagerung und Führung des Kolbens (16) eine integrierte Blattfederkonstruktion (19, 20a, 20b, 20c, 21a, 21b, 21c) ist.

15. Vorrichtung nach Anspruch 14, dadurch gekennzeichnet, daß zumindest der Kolben (16) und die Kammer (17) ganz oder zumindest an der Oberfläche aus einem Material bestehen, welches aus einem gegenüber der durchfließenden Flüssigkeit bzw. des durchfließenden Gases resistenten Material besteht.

16. Vorrichtung nach einem der Ansprüche 13 bis 15, dadurch gekennzeichnet, daß der Kolben (16) in der durchflußbegrenzenden Vorrichtung (6.1, 6.2, 6a.1, 6a.2, 15, 42) drei stabile Lagezustände und daß die Kammer (17) zumindest einen seitlichen Zufluß (25) und mindestens zwei sich stirnseitig gegenüberliegende zentrische Abflüsse (30a, 30b) besitzt.

17. Vorrichtung nach einem der Ansprüche 12 bis 16, dadurch gekennzeichnet, daß der Kolben (16) ein Permanentmagnet ist und daß stirnseitig auf die Kammer (17) je ein separat ansteuerbarer Elektromagnet (27a, 27b) mit einem Spulenkörper (28a, 28b) aufgesetzt ist.

18. Vorrichtung nach einem der Ansprüche 12 bis 17, dadurch gekennzeichnet, daß mehrere tristabile durchflußbegrenzende Vorrichtungen (6.1, 6.2, 6a.1, 6a.2, 15, 42) hintereinander, parallel oder in Kombination geschaltet sind.

19. Vorrichtung nach einem der Ansprüche 12 bis 17, dadurch gekennzeichnet, daß die Vorrichtung (6.1, 6.2, 6a.1, 6a.2, 15, 42) in einer Infusionspumpe (1, 33) zur Implantation in den Körper eines Patienten zur medikamentösen Behandlung eines Patienten eingebaut ist, daß die Infusionspumpe (1, 33) einen Vorratsbehälter (3, 48) zum Speichern eines gewählten Medikaments besitzt, daß eine flüssigkeitsfördernde Einrichtung (5, 50) zum Transport des Medikaments vom Vorratsbehälter (3, 48) zu der durchflußbegrenzenden Vorrichtung (6.1, 6.2, 6a.1, 6a.2, 15, 42) angebracht ist und daß mindestens eine Einleitung (26a, 26b, 51, 52) des aus dem Vorratsbehälter (3, 48) kommenden, in diesem gespeicherten, ausgewählten Medikaments in den Körper aufweist.

20. Vorrichtung nach Anspruch 19, dadurch gekennzeichnet, daß die flüssigkeitsfördernde Einrichtung (5, 50) aus einem Gasdruckbehälter besteht, welcher auf den Vorratsbehälter (3, 48) einen Druck in Richtung der durchflußbegrenzenden Vorrichtung (6.1, 6.2, 6a.1, 6a.2, 15, 42) ausübt.
